# EUROPÄISCHE PATENTSCHRIFT

(11) **EP 0 665 212 B1**
(45) Veröffentlichungstag und Bekanntmachung des Hinweises auf die Patenterteilung: **09.06.1999**
(21) Anmeldenummer: 95100486.0
(22) Anmeldetag: 16.01.1995
(51) Int. Cl.: C07C 67/343, C07C 69/612, C07C 69/614, C07C 51/08, C07C 27/02, C07C 57/30, C07C 57/32, C07C 255/33, C07C 253/30

(54) **Verfahren zur Herstellung von 2,4,6-Trimethylphenylessigsäure**
Process for the preparation of 2,4,6-trimethylphenylacetic acid
Procédé pour la préparation d'acide triméthylphénylacétique

(30) Priorität: 27.01.1994 DE 4402403
(43) Veröffentlichungstag der Anmeldung: 02.08.1995
(73) Patentinhaber: BAYER AG, 51368 Leverkusen (DE)
(72) Erfinder: Stelzer, Dr. Uwe, D-51381 Leverkusen (DE)

(56) Entgegenhaltungen:
- EP-A- 0 220 947
- EP-A- 0 310 186
- EP-A- 0 377 893
- EP-A- 0 410 590
- BE-A- 633 761
- FR-A- 1 374 665
- GB-A- 971 700
- US-A- 4 707 549
- CHEMICAL ABSTRACTS, vol. 107, no. 21, 1987 Columbus, Ohio, US; abstract no. 197813z, & JP-A-62 126 159 (KYOWA HAKKO KOGYO CO LTD)
- BULL. CHIM. SOC. FR., no.9-10, 1948 Seiten 995-1001, J. LICHTENBERGER ET AL
- CHEMICAL ABSTRACTS, vol. 91, no. 3, 1979 Columbus, Ohio, US; abstract no. 19832y, & IZV. AKAD. NAUK SSSR, SER. KHIM., no.4, 1979 Seiten 806-809, A.V. FOKIN ET AL
- CHEMICAL ABSTRACTS, vol. 89, no. 3, 1978 Columbus, Ohio, US; abstract no. 168303h, & JP-A-53 149 945 (MITSUI TOATSU CHEMICALS INC)
- CHEMICAL ABSTRACTS, vol. 90, no. 21, 1979 Columbus, Ohio, US; abstract no. 23975y, & JP-A-53 012 837 (MITSUI TOATSU CHEMICALS INC)
- TETRAHEDRON LETT., Bd. 32, Nr. 41, 1991, Seiten 5741 - 5744 C.C. SILVE ET AL.
- J. AM. CHEM. SOC., Bd. 74, 1952, Seiten 6246 - 6250 D.A.MCCAULEY

## Beschreibung

Die Erfindung betrifft ein neues Verfahren zur Herstellung von 2,4,6-Trimethylphenylessigsäure.

Aus JP-A-62 126 159 ist ein Verfahren zur Herstellung von Phenylessigsäurenitrilen durch Umsetzung von Benzolderivaten mit sulfonyloxyaktivierten Alkylnitrilen in Gegenwart einer Lewissäure bekannt. Die erzielten Ausbeuten sind jedoch nicht befriedigend.

Es ist ferner bekannt, daß man substituierte Phenylessigsäuren und ihre Derivate erhält, wenn man entsprechende substituierte Aromaten durch eine Chlormethylierung oder Brommethylierung in die Chlormethyl- oder Brommethylaromaten überführt, diese dann mit Cyaniden umsetzt und sie anschließend zur Säure verseift (vgl. z.B. J. Org. Chem. 58, 1262 (1993), Houben-Weyl, Methoden der Organischen Chemie, Georg Thieme Verlag, Stuttgart, Band 5/4 Seite 484, 1960, Houben-Weyl, Methoden der Organischen Chemie, Georg Thieme Verlag, Stuttgart, Band VIII, Seite 427, 1952). Dieses Verfahren weist jedoch den Nachteil auf, daß wegen der Möglichkeit, daß bei der Halogenmethylierung kanzerogene Bishalogenmethylether entstehen, ein erhöhter Aufwand an Sicherheitsvorkehrungen bei der Reaktionsdurchführung erforderlich ist.

In der Publikation J. Org. Chem. 1991, 56, 183-187 wird die Herstellung von 2-Arylpropionsäureestern und 2-Arylbuttersäureestern durch Alkylierung von aromatischen Verbindungen mit den entsprechenden 2-(Sulfonyloxy)propionsäureestern bzw. 2-(Sulfonyloxy)buttersäureestern in Gegenwart von AlCl₃ beschrieben. Die Synthese von in α-Stellung unsubstituierten Phenylessigsäuren wird nicht beschrieben (vgl. z.B. auch Org. Prep. Proc. Int. 1989, 21 (3), 375-376, J. Amer. Chem. Soc. 113, 9630 (1991), Tetrahedron Lett, 26, 477 (1985), J. Med. Chem. 24, 382 (1981), J. Amer. Chem. Soc. 95, 3340 (1973).

Weiter ist bekannt, daß man Phenylessigsäuren durch Carboxylierung von Benzylhalogeniden unter Phasentransferbedingungen erhält (Tetrahedron Lett., 24 (37), 4005-4008 (1983); J. Chem. Soc., Chem. Commun., (24), 1090-1091 (1978). Ein erheblicher Nachteil dieser Verfahren ist neben dem Einsatz von Eisen- und Cobaltcarbonylen, daß sie zum Teil unter Druck durchgeführt werden müssen und zu Reaktionsgemischen führen.

Gegenstand der vorliegenden Erfindung ist ein Verfahren zur Herstellung von 2,4,6-Trimethylphenylessigsäure der Formel (I) 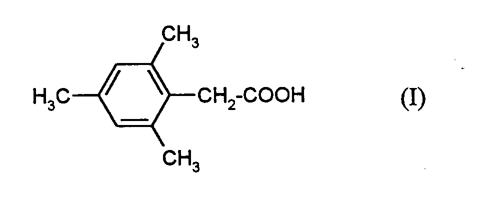 dadurch gekennzeichnet, daß man sulfonyloxyaktivierte Hydroxyessigsäurederivate der Formel (II)

R³-SO₂-O-CH₂-R¹ (II)

in welcher
- R¹: für Cyano, Carboxyl, Methoxycarbonyl, Ethoxycarbonyl oder n-Butyloxycarbonyl steht und
- R³: für Halogen, Alkyl, Halogenalkyl oder jeweils gegebenenfalls substituiertes Aryl oder Benzyl steht,
mit dem Aromaten der Formel (III) 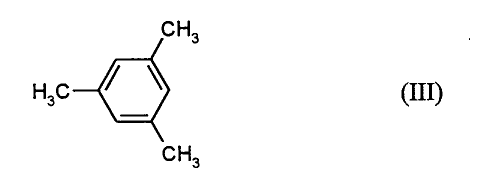 gegebenenfalls in Gegenwart eines Verdünnungsmittels und in Gegenwart von 2,3 bis 4,0 Mol Aluminiumchlorid pro Mol der Verbindung der Formel (II) umsetzt und gegebenenfalls anschließend hydrolysiert.

Es ist als ausgesprochen überraschend anzusehen, daß nach dem erfindungsgemäßen Verfahren die 2,4,6-Trimethylphenylessigsäure der Formel (I) in sehr guten Ausbeuten und in hoher Reinheit durch eine Friedel-Crafts-Alkylierung mit Sulfonyloxyessigsäurederivaten erhalten werden, da üblicherweise unter diesen Reaktionsbedingungen mit einem höheren Anteil an unerwünschten Nebenprodukten und damit verbunden schlechteren Ausbeuten zu rechnen ist. Ein weiterer Vorteil des erfindungsgemäßen Verfahrens ist, daß die Bildung des stark kanzerogenen Bishalogenmethylethers, der anfällt, wenn Phenylessigsäurederivate über die Zwischenstufe der Chlormethylierung von Aromaten hergestellt werden, vermieden wird. Das neue Verfahren kann somit unter deutlich verbesserten Sicherheits- und Umweltaspekten durchgeführt werden.

Das erfindungsgemäße Verfahren erweist sich im Vergleich mit vorbekannten Methoden auch als ein wesentlich kostengünstigerer Herstellungsweg für 2,4,6-Trimethylphenylessigsäure der Formel (I) und stellt somit eine wertvolle Bereicherung des Standes der Technik dar.

Verwendet man beispielsweise Methylsulfonyloxyessigsäure-n-butylester als Ausgangsstoff, so läßt sich der Reaktionsablauf des erfindungsgemäßen Verfahrens durch das folgende Formelschema darstellen:

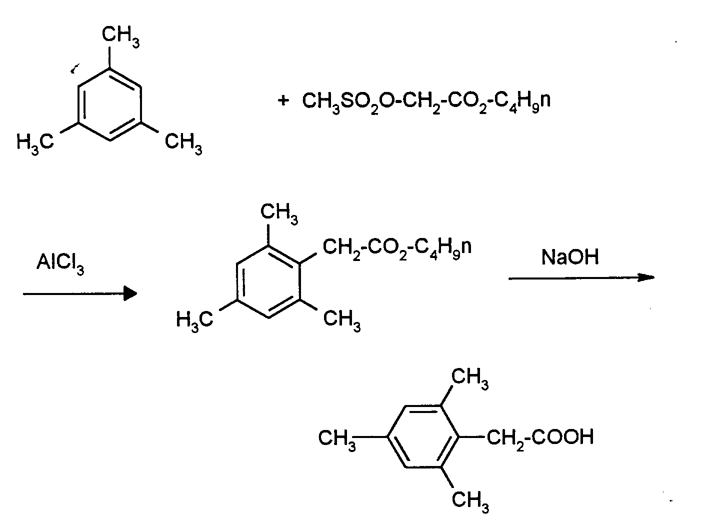

Die bei dem erfindungsgemäßen Verfahren zur Herstellung der Verbindung der Formel (I) als Ausgangsstoffe zu verwendenden sulfonyloxyaktivierten Hydroxyessigsäurederivate sind durch die Formel (II) allgemein definiert. In der Formel (II) steht R³ vorzugsweise für Chlor, Brom, C₁-C₈-Alkyl, C₁-C₈-Halogenalkyl oder für jeweils gegebenenfalls einfach bis dreifach gleich oder verschieden durch Cyano, Nitro, Halogen, C₁-C₈-Alkyl, C₁-C₈-Akoxy oder C₁-C₈-Halogenalkoxy substituiertes Phenyl oder Benzyl.

R³ steht besonders bevorzugt für Chlor, Brom, für gegebenenfalls einfach oder mehrfach durch Fluor, Chlor und/oder Brom substituiertes Methyl, Ethyl, n- oder i-Propyl, n-, s-, i- oder t-Butyl oder für gegebenenfalls einfach bis dreifach gleich oder verschieden durch Cyano, Nitro, Chlor oder Brom oder durch gegebenenfalls einfach oder mehrfach durch Fluor, Chlor und/oder Brom substituiertes Methyl, Ethyl, n- oder i-Propyl, n-, s-, i- oder t-Butyl substituiertes Phenyl.

Einige der sulfonyloxyaktivierten Hydroxyessigsäurederivate der Formel (II) sind bereits prinzipiell aus der Literatur bekannt (vgl. z.B. DE-A-2 431 734, JP-A-59 010 564, JP-A-59 010 564, US-A-4 260 555, Bull. Chem. Soc. Jpn. 43, 1572-1573, (1970), J. Phys. Chem. 94, 8835-8839, (1990), J. Org. Chem. 42, 3109-3113, (1977), Izv. Akad. Nauk SSSR, Ser. Khim (7), 1682, 1983) und können nach den dort beschriebenen Verfahren erhalten werden.

Der Aromat der Formel (III) ist eine bekannte Synthesechemikalie.

Das erfindungsgemäße Verfahren wird vorzugsweise in Gegenwart eines Verdünnungsmittels durchgeführt.

Als Verdünnungsmittel zur Durchführung des Verfahrens kommen alle unter den Reaktionsbedingungen inerten, üblichen organischen Lösungsmittel in Betracht. Hierzu gehören beispielsweise insbesondere inerte Solventien wie Kohlenwasserstoffe, Halogenkohlenwasserstoffe, Nitroalkane und Nitroaromaten. Genannt seien Hexan, Cyclohexan, Dichlorethan, Chloroform, Tetrachlorkohlenstoff, Nitromethan oder Nitrobenzol. Besonders vorteilhaft ist es, den für die Umsetzung gemäß dem erfindungsgemäßen Verfahren benötigten Aromaten der Formel (III) selbst als Verdünnungsmittel einzusetzen.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise bei Temperaturen zwischen 20°C und 150°C.

Das erfindungsgemäße Verfahren wird im allgemeinen unter Normaldruck durchgeführt. Es ist jedoch auch möglich, unter erhöhtem oder vermindertem Druck zu arbeiten.

Die erfindungsgemaße Verfahrensstufe wird in Gegenwart von Aluminiumchlorid durchgeführt.

Auf beispielsweise 1 Mol der sulfonyloxyaktivierten Hydroxyessigsäurederivate der Formel (II) werden 2,3 bis 4,0 Mol, besonders bevorzugt 2,75 bis 4,0 Mol Aluminiumchlorid und 1 bis 20 Mol des Aromaten in 50 bis 500 ml in einem der oben genannten Verdünnungsmittel eingesetzt.

In einer bevorzugten Ausführungsform wird das erfindungsgemäße Verfahren so durchgeführt, daß der Aromat der Formel (III) selbst als Verdünnungsmittel oder in einem der oben angegebenen Lösungsmittel mit Aluminiumchlorid vorgelegt wird, danach mit dem sulfonyloxyaktivierten Hydroxyessigsäurederivat der Formel (II) versetzt wird und danach bei der angegebenen Temperatur so lange gerührt wird, bis die Reaktion beendet ist.

Je nach Ausgangsstoffen und den gewählten Reaktionsbedingungen können auf diese Weise entweder 2,4,6-Trimethylphenylessigsäureester, die 2,4,6-Trimethylphenylessigsäure oder das 2,4,6-Trimethylphenylessigsäurenitril erhalten werden.

In einer weiteren Ausführungsform wird das erfindungsgemäße Verfahren durchgeführt, indem man die sulfonyloxyaktivierten Hydroxyessigsäurederivate der Formel (II) und den Aromaten der Formel (III) gegebenenfalls in einem Lösungsmittel vorlegt, danach mit Aluminiumchlorid versetzt und bei der angegebenen Temperatur bis zum Reaktionsende nachrührt.

Die Aufarbeitung kann nach üblichen Methoden erfolgen, beispielsweise durch Hydrolysieren mit 10%iger Salzsäure, Extraktion mit einem organischen, mit Wasser praktisch nicht mischbaren Lösungsmittel, Trocknen der organischen Phase und Entfernen des Lösungsmittels unter vermindertem Druck. Das so erhaltene Rohprodukt kann durch Umkristallisation, Chromatographie oder Destillation gereinigt werden.

Das nach dem oben beschriebenen Verfahren gegebenenfalls erhaltene Rohprodukt (Ester oder Nitril) wird durch saure oder alkalische Hydrolyse zu 2,4,6-Trimethylphenylessigsäure verseift. Die Verseifung erfolgt nach allgemein bekannten Methoden (vgl. z.B. Houben-Weyl, Methoden der Organischen Chemie, Georg Thieme Verlag, Stuttgart, Band VIII, Seite 427, und Bl. Soc. Chim. Belg., 64, Seite 91, (1954), J. Am. Chem. Soc. 72, 4091 (1950), Org. Synth. Coll. Vol. III 557, 558 (1955)), indem man beispielsweise 1 Mol des nach dem oben beschriebenen Verfahren erhaltenen Rohprodukts in eine Lösung von 1 bis 50 Mol Natriumhydroxyd in Methanol/Wasser gibt und mehrere Stunden bei Temperaturen zwischen 20°C und 150°C rührt. Zur Aufarbeitung wird das Reaktionsgemisch in einem praktisch mit Wasser nicht mischbaren Lösungsmittel extrahiert, die wäßrige Phase mit Mineralsäure angesäuert und das Produkt abfiltriert oder extrahiert.

Es kann jedoch auch von Vorteil sein, das nach dem oben beschriebenen Verfahren erhaltene Rohprodukt (Ester oder Nitril) mit Mineralsäuren, wie beispielsweise konzentrierter Schwefelsäure oder Salzsäure in Wasser bei Temperaturen zwischen 20°C und 150°C umzusetzen. Das Produkt wird durch Filtration oder Extraktion isoliert.

Die Reinheit des nach der oben beschriebenen sauren oder alkalischen Verseifung der Rohprodukte der Formel (I) erhaltenen Endprodukts kann gegebenenfalls durch übliche Methoden, wie beispielsweise Destillation, Chromatographie oder Kristallisation weiter erhöht werden.

Die nach dem erfindungsgemäßen Verfahren herzustellende 2,4,6-Trimethylphenylessigsäure der Formel (I) kann als Ausgangsstoff zur Herstellung von Herbiziden und Schädlingsbekämpfungsmitteln verwendet werden (vgl. z.B. EP-A-528 156).

### Herstellungsbeispiele

### Beispiel II-1 CH₃SO₂OCH₂COOCH₃

18 g (0,2 Mol) Hydroxyessigsäuremethylester werden in 250 ml Dichlormethan vorgelegt und auf 0°C gekühlt. Zu dieser Lösung gibt man 20,2 g (0,2 Mol) Triethylamin und tropft bei 0°C über einen Zeitraum von 1 Stunde 34,3 g (0,3 Mol) Methansulfonsäurechlorid zu. Man rührt bei 0°C über Nacht, versetzt den Ansatz mit Eiswasser, extrahiert die wäßrige Phase mehrmals mit Dichlormethan und trocknet die vereinigten organischen Phasen über Natriumsulfat. Nach der Filtration des Trockenmittels und dem Abdestillieren des Lösungsmittels erfolgt eine Destillation im Feinvakuum. Man isoliert 29,7 g (88 % der Theorie) Methylsulfonyloxyessigsäuremethylester als farbloses Öl mit einem Gehalt >98 % (GC) bei einem Siedepunkt von 98° bis 101°C/0,1 mbar.

### Beispiel II-a-1 CH₃SO₂OCH₂COO-nC₄H₉

27,8 g (0,2 Mol) Hydroxyessigsäure-n-butylester (95 % GC) und 20,2 g (0,2 Mol) Triethylamin werden bei -5°C in 300 ml Dichlormethan vorgelegt. Bei 0° bis 5°C werden 34,4 g (0,3 Mol) Methansulfonsäurechlorid in 50 ml Dichlormethan langsam zugetropft. Man rührt über Nacht bei 0°C nach, versetzt den Ansatz mit Eiswasser, trennt die Phasen und extrahiert die wäßrige Phase noch zweimal mit Dichlormethan. Die vereinigten organischen Phasen werden getrocknet und das Lösungsmittel am Rotationsverdampfer entfernt. Zurück bleibt ein farbloses Öl, welches im Vakuum andestilliert wird. Man isoliert Methylsulfonyloxyessigsäure-n-butylester als ein nahezu farbloses Öl (40,5 g, 96,3 % der Theorie, Gehalt 91 % GC).
¹H-NMR* (δ = 0,946 (t, 3H, CH₃), 1,33-1,45 (m, 2H, CH₂), 1,61-1,706 (m, 2H, CH₂), 3,21 (s, 3H, CH₃), 4,22 (t, 2H, CH₂), 4,77 (s, 2H, CH₂)).

*) Die ¹H-NMR-Spektren wurden in Deuterochloroform (CDCl₃) mit Tetramethylsilan (TMS) als innerem Standard aufgenommen. Angegeben ist die chemische Verschiebung als δ-Wert in ppm.

### Beispiel II a-2

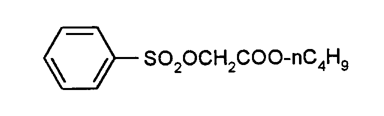

Auf analoge Weise werden aus 27,8 g (0,2 Mol) Hydroxyessigsäure-n-butylester, 46 g (0,26 Mol) Benzolsulfonsäurechlorid und 20,2 g (0,2 Mol) Triethylamin 45,8 g (84 % der Theorie) Phenylsulfonyloxyessigsäure-n-butylester erhalten.
¹H-NMR* (δ = 0,83 (t, 3H, CH₃), 1,19-1,31 (m, 2H, CH₂), 1,45-1,55 (m, 2H, CH₂), 4,05 (t, 2H, CH₂), 4,54 (s, 2H, CH₂), 7,47-7,89 (m, 5H, Phenyl)).

*) Die ¹H-NMR-Spektren wurden in Deuterochloroform (CDCl₃) mit Tetramethylsilan (TMS) als innerem Standard aufgenommen. Angegeben ist die chemische Verschiebung als δ-Wert in ppm.

### Beispiel I-1

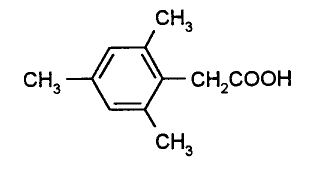

39,9 g (0,3 Mol) Aluminiumchlorid werden in 200 ml Mesitylen vorgelegt und auf 40 bis 45°C erwärmt. Man rührt 0,5 Stunden nach und tropft bei der angegebenen Temperatur 22,0 g (0,1 Mol) Methylsulfonyloxyessigsäure-n-butylester (Gehalt 91 % GC, 0,095 Mol) über einen Zeitraum von 1 Stunde zu. Über Nacht wird bei 40 bis 45°C nachgerührt und mit 10%iger Salzsäure hydrolysiert. Die organische Phase wird abgetrennt, die wäßrige Phase zweimal mit Diethylether extrahiert, vereinigt und getrocknet. Nach dem Abziehen des Lösungsmittels und des Mesitylens wird das erhaltene ölige Rohprodukt mit 12 g NaOH in 25 ml Wasser und 80 ml Methanol versetzt und 5 Stunden am Rückfluß erhitzt. Die Reaktionslösung wird auf Wasser gegossen, zweimal mit Diethylether extrahiert und die wäßrige Phase mit konzentrierter Salzsäure angesäuert. Durch mehrmalige Extraktion mit Methylenchlorid und Vereinigen und Trocknen der organischen Phasen isoliert man nach dem Abziehen des Lösungsmittels 14,0 g Mesitylessigsäure (82,7 % der Theorie, Gehalt 96 % GC).

### Beispiel I-2

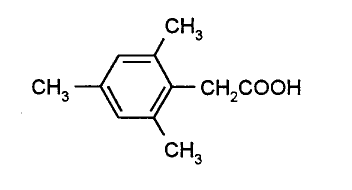

79,8 g (0,6 Mol) Aluminiumchlorid werden in 400 ml Mesitylen vorgelegt und auf 40 bis 45°C erwärmt. Man rührt 1 Stunde nach und tropft bei der angegebenen Temperatur 33,6 g (0,2 Mol) Methylsulfonyloxyessigsäuremethylester (98 %, GC) über einen Zeitraum von 2 bis 3 Stunden zu. Über Nacht wird bei 40 bis 45°C nachgerührt und mit 10%iger Salzsäure hydrolysiert. Die organische Phase wird abgetrennt und die wäßrige Phase zweimal mit Diethylether oder Dichlormethan extrahiert, vereinigt und getrocknet. Nach dem Abziehen des leichter flüchtigen Lösungsmittels erhält man 38,7 g des öligen Rohprodukts. Dieses kann zur Isolierung des Esters destilliert oder mit 25 g NaOH in 50 ml Wasser und 160 ml Methanol unter Rückfluß (5 Stunden) in die Mesitylessigsäure überführt werden. Die Reaktionslösung wird auf Wasser gegossen, zweimal mit Diethylether extrahiert und anschließend die wäßrige Phase mit konzentrierter Salzsäure angesäuert. Das ausgefallene Produkt wird abgesaugt. Durch mehrmalige Extraktion mit Dichlormethan, Vereinigen und Trocknen der organischen Phasen isoliert man nach dem Abziehen des Lösungsmittels 30 g Mesitylessigsäure (84,2 % der Theorie, Gehalt 96 % GC).

### Beispiel I-3

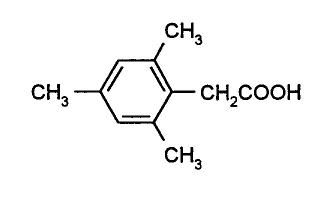

17,8 g (0,133 Mol) Aluminiumchlorid werden in 100 ml Mesitylen vorgelegt und auf 40 bis 45°C erwärmt. Über einen Zeitraum von 1 bis 2 Stunden werden 10 g (0,044 Mol) p-Toluolsulfonyloxyessigsäuremethylester zugetropft. Man rührt über Nacht bei 40 bis 45°C nach, hydrolysiert mit 100 ml 10%iger Salzsäure, trennt die organische Phase ab, extrahiert die wäßrige Phase noch zweimal mit Methylenchlorid und vereinigt und trocknet die organischen Phasen. Nach dem Abdestillieren des Lösungsmittels wird das ölige Rohprodukt direkt mit 6 g NaOH in 20 ml Wasser und 50 ml Methanol 3 Stunden am Rückfluß erhitzt und gerührt. Die Reaktionslösung wird auf Wasser gegossen, zweimal mit Diethylether extrahiert und anschließend die wäßrige Phase mit konzentrierter Salzsäure angesäuert. Durch dreimalige Extraktion mit Dichlormethan, Vereinigen und Trocknen der organischen Phase isoliert man nach dem Abziehen des Lösungsmittels 6,6 g (83 % der Theorie) Mesitylessigsäure.

### Beispiel I-4

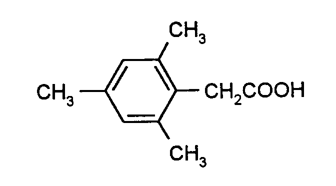

8,0 g (0,06 Mol) und 5,4 g (0,0198 Mol) Phenylsulfonyloxyessigsäure-n-butylester werden analog Beispiel 7 in 60 ml Mesitylen umgesetzt und mit 5 g NaOH in 15 ml Wasser und 50 ml Methanol zur Säure verseift. Man isoliert 3,0 g (85 % der Theorie) an Mesitylessigsäure.

### Beispiel Ia-1

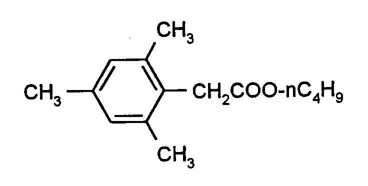

In 100 ml Mesitylen werden 20 g (0,15 Mol) Aluminiumchlorid vorgelegt und auf 40 bis 45°C erwärmt. Über den Zeitraum von einer Stunde werden 10,6 g (0,05 Mol) Methylsulfonyloxyessigsäure-n-butylester zugetropft. Man rührt über Nacht nach und hydrolysiert mit 100 ml 10%iger Salzsäure, trennt die organische Phase ab und extrahiert die wäßrige Phase mit Methylenchlorid. Die organische Phase wird getrocknet und das Lösungsmittel abgetrennt. Das erhaltene Rohprodukt wird mehrmals mit Petrolether versetzt und vom zurückbleibenden farblosen Feststoff abgetrennt. Auf diese Weise isoliert man direkt 1,3 g (14 % der Theorie) an Mesitylessigsäure. Die Petroletherphase wird eingeengt und das Rohprodukt im Vakuum destilliert (0,01 mbar, Sdp. 120°C). Man isoliert 8,8 g (75,1 % der Theorie) an Mesitylessigsäure-n-butylester.
¹H-NMR* (δ = 0,90 (t, 3H, CH₃), 1,29-1,37 ( m, 2H, CH₂), 1,54-1,61 (m, 2H, CH₂), 2,25 (s, 3H, CH₃), 2,29 (s, 6H, 2x CH₃), 3,63 (s, 2H, CH₂), 4,07 (t, 2H, CH₂), 6,85 (m, 2H, aromat. Protonen)).

*) Die ¹H-NMR-Spektren wurden in Deuterochloroform (CDCl₃) mit Tetramethylsilan (TMS) als innerem Standard aufgenommen. Angegeben ist die chemische Verschiebung als δ-Wert in ppm.

### Beispiel I-5

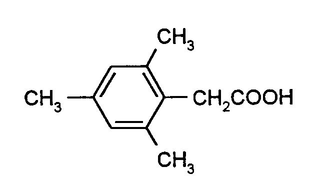

In 40 ml Mesitylen werden 8,0 g (0,06 Mol) Aluminiumchlorid vorgelegt, auf 40 bis 45°C erwärmt und mit 4,1 g (0,02 Mol) Phenylsulfonyloxyethannitril versetzt und bei 40 bis 45°C 6 Stunden gerührt. Danach werden 100 ml 10%iger Salzsäure zugegeben und das Reaktionsgemisch mit Methylenchlorid mehrmals extrahiert. Die organischen Phasen werden getrocknet. Das nach dem Abziehen des Lösungsmittels erhaltene Rohprodukt wird mit 9 ml konzentrierter Schwefelsäure in 11 ml Wasser 6 Stunden unter Rückfluß erhitzt, mit Eiswasser versetzt, mit NaOH alkalisch gestellt und mit Diethylether extrahiert. Die wäßrige Phase wird mit konzentrierter Salzsäure sauer gestellt und dreimal mit Methylenchlorid extrahiert und die organische Phase getrocknet. Nach dem Abdestillieren des Lösungsmittels isoliert man 2,3 g (62 % der Theorie) Mesitylessigsäure.

### Beispiel I-6

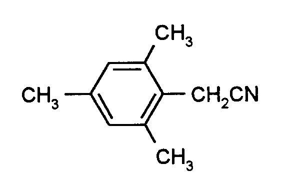

In 40 ml Mesitylen werden 8,0 g (0,06 Mol) Aluminiumchlorid bei 0°C vorgelegt. Danach werden 4,1 g (0,02 Mol) Phenylsulfonyloxyethannitril zugegeben und das Reaktionsgemisch über Nacht bei Raumtemperatur gerührt. Das nach der Hydrolyse erhaltene Rohprodukt wird an Kieselgel mit Dichlormethan chromatographiert. Man isoliert 2,3 g (72,3 % der Theorie) 2,4,6-Trimethylbenzylcyanid.

## Patentansprüche

1. Verfahren zur Herstellung von 2,4,6-Trimethylphenylessigsäure der Formel (I) 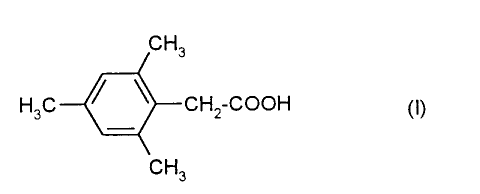 dadurch gekennzeichnet, daß man sulfonyloxyaktivierte Hydroxyessigsäurederivate der Formel (II)
R³-SO₂-O-CH₂-R¹ (II)
in welcher
R¹ für Cyano, Carboxyl, Methoxycarbonyl, Ethoxycarbonyl oder n-Butyloxycarbonyl steht und
R³ für Halogen, Alkyl, Halogenalkyl oder jeweils gegebenenfalls substituiertes Aryl oder Benzyl steht,
mit dem Aromaten der Formel (III) 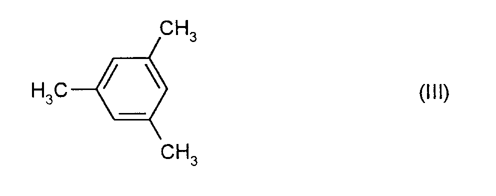 gegebenenfalls in Gegenwart eines Verdünnungsmittels und in Gegenwart von 2,3 bis 4,0 mol Aluminiumchlorid pro mol der Verbindung der Formel (II) umsetzt und gegebenenfalls anschließend hydrolysiert.

2. Verfahren gemäß Anspruch 1, dadurch gekennzeichnet, daß 2,75 bis 4,0 mol Aluminiumchlorid pro mol der Verbindung der Formel (II) eingesetzt werden.

## Claims

1. Process for preparing 2,4,6-trimethylphenylacetic acid of the formula (I) 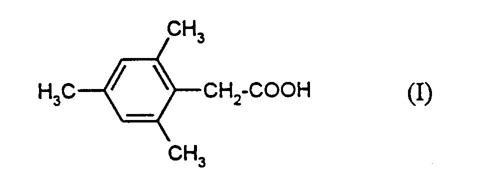 characterized in that sulphonyloxy-activated hydroxyacetic acid derivatives of the formula (II)
R³-SO₂-O-CH₂-R¹ (II)
in which
R¹ represents cyano, carboxyl, methoxycarbonyl, ethoxycarbonyl or n-butyloxycarbonyl and
R³ represents halogen, alkyl, halogenoalkyl, or represents in each case substituted or unsubstituted aryl or benzyl
are reacted with the aromatic of the formula (III) 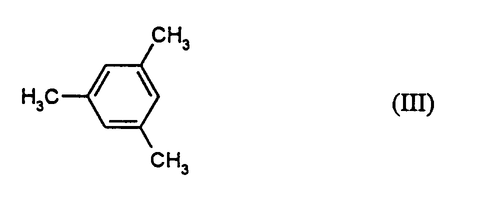 if desired in the presence of a diluent and in the presence of 2.3 to 4.0 mol of aluminium chloride per mole of the compound of the formula (II), and then, if desired, the resulting product is hydrolysed.

2. Process according to Claim 1, characterized in that 2.75 to 4.0 mol of aluminium chloride are used per mole of the compound of the formula (II).

## Revendications

1. Procédé de production d'acide 2,4,6-triméthylphénylacétique de formule (I) 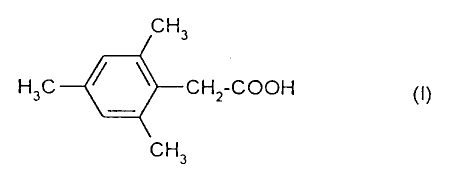 caractérisé en ce qu'on fait réagir des dérivés d'acide hydroxyacétique activés par un groupe sulfonyloxy, de formule (II)
R³-SO₂-O-CH₂-R¹ (II)
dans laquelle
R¹ est un groupe cyano, carboxyle, méthoxycarbonyle, éthoxycarbonyle ou n-butyloxycarbonyle et
R³ est un halogène, un groupe alkyle, halogénalkyle, ou un groupe aryle ou benzyle portant chacun le cas échéant un substituant,
avec le composé aromatique de formule (III) 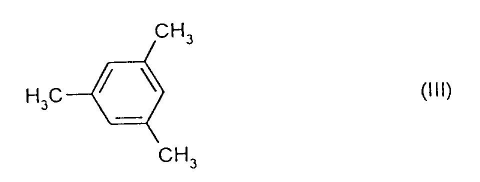 le cas échéant en présence d'un diluant et en présence de 2,3 à 4,0 moles de chlorure d'aluminium par mole de composé de formule (II) et on effectue ensuite éventuellement une hydrolyse.

2. Procédé suivant la revendication 1, caractérisé en ce qu'on utilise 2,75 à 4,0 moles de chlorure d'aluminium par mole de composé de formule (II).
